# FASCICULE DE BREVET EUROPEEN

(11) **EP 1 474 079 B1**
(45) Date de publication et mention de la délivrance du brevet: **19.03.2008**
(21) Numéro de dépôt: 03717398.6
(22) Date de dépôt: 13.02.2003
(51) Int. Cl.: A61F 2/38

(54) **PROTHESE TOTALE DU GENOU**
KNIEGELENKSTOTALPROTHESE
COMPLETE KNEE PROSTHESIS

(30) Priorité: 14.02.2002 FR 0201862
(43) Date de publication de la demande: 10.11.2004
(73) Titulaire: Afriat, Jacques, 11100 Narbonne (FR)
(72) Inventeur: Afriat, Jacques, 11100 Narbonne (FR)
(74) Mandataire: Jeannet, Olivier
(86) Numéro de dépôt international: PCT/FR2003/000466
(87) Numéro de publication internationale: WO 2003/068109

(56) Documents cités:
- EP-A- 0 567 705
- FR-A- 2 805 456
- US-A- 4 209 861
- US-A- 5 011 496
- US-A- 5 370 699

## Description

La présente invention concerne une prothèse totale du genou.

Une telle prothèse comprend, comme cela est bien connu, un élément fémoral reproduisant les condyles fémoraux, une embase tibiale formant une plaque supérieure d'appui et un plateau intermédiaire en matériau favorisant le glissement, notamment en polyéthylène à haute densité. Ce plateau intermédiaire présente une face inférieure destinée à prendre appui contre la plaque supérieure d'appui de l'embase tibiale et des cavités glénoïdes recevant les condyles de l'élément fémoral.

Dans une prothèse totale de genou, il est souhaitable que les condyles prothétiques reproduisent globalement la forme des condyles naturels d'une articulation du genou, pour l'obtention d'une bonne reproduction du mouvement de cette articulation et d'une tension ligamentaire adéquate. Ces condyles prothétiques doivent donc présenter, vus dans le plan sagittal, une forme spiroïdale correspondant sensiblement à celle des condyles naturels, c'est-à-dire avec une réduction progressive de leurs rayons en direction des secteurs postérieurs.

Il résulte de cette réduction de rayons que la surface de contact entre les condyles et le plateau intermédiaire se modifie au cours du mouvement de flexion ou d'extension de l'articulation.

Certaines prothèses privilégient la congruence en extension, soit entre 0 et 40° à 60° environ de flexion. Dans ce cas, le contact des condyles prothétiques avec le plateau intermédiaire, au-delà de ces angles de flexion, devient linéaire ou même ponctuel, et non surfacique, ce qui rend possible l'avancée du fémur par rapport au tibia, selon de mouvement dit "de tiroir postérieur". Ce mouvement est contraire au mouvement physiologique de recul du fémur par rapport au tibia pendant la flexion, dénommé "roll-back".

Il est souhaitable de reproduire ce mouvement de "roll-back" sur une prothèse de genou. En effet, ce mouvement permet d'augmenter la force d'extension du quadriceps, en augmentant le bras de levier de celui-ci. De plus, ce mouvement permet de retarder la butée du fémur contre le plateau intermédiaire en fin de flexion et de réduire l'effet de came des parties molles dans cette même position, ce qui augmente l'amplitude de mouvement de l'articulation et limite les sollicitations sur les interfaces d'ancrage osseux de l'élément fémoral et de l'embase tibiale.

Pour résoudre le problème de mouvement "de tiroir postérieur", il est connu d'adjoindre à une prothèse de genou un système de "postéro-stabilisation", qui consiste une butée entre une barre ou un bossage aménagé sur l'élément fémoral, entre les deux condyles, et une éminence médiane du plateau intermédiaire. Ce système limite le mouvement d'avancée du fémur par rapport au tibia mais la surface de contact entre le fémur et l'élément intermédiaire est nécessairement linéaire ou ponctuelle en flexion. Cette surface de contact limitée est une source d'usure du plateau intermédiaire et est donc préjudiciable à la pérennité de celui-ci.

Certaines prothèses permettent une congruence surfacique en extension et flexion : le rayon condylien sagittal postérieur s'inscrit dans un cercle, et le plateau intermédiaire présente des surfaces glénoïdes à profil circulaire correspondant. Dans ce cas, le contact surfacique est maintenu tout au long de la flexion, mais, compte tenu de cette congruence, les mouvements de translation du fémur par rapport aux plateaux intermédiaires sont limités ou impossibles.

Certaines prothèses permettent une mobilité antéro-postérieure entre le plateau intermédiaire et l'élément tibial. Dans ce cas, le mouvement du fémur par rapport au tibia résulte non pas de la forme des surfaces de contact mais de l'action des muscles et des ligaments. Le mouvement précité de "roll-back" étant essentiellement contrôlé par le ligament croisé postérieur, ces prothèses imposent la conservation de ce ligament croisé postérieur, ce qui n'est pas toujours possible, souhaité ou souhaitable.

La présente invention vise à remédier aux inconvénients précités des prothèses totales du genou existantes, en fournissant une prothèse à "postéro-stabilisation" permettant de conserver une congruence élevée entre l'élément fémoral et le plateau intermédiaire sur l'ensemble du mouvement de l'articulation, tout en permettant un mouvement de "roll-back" parfaitement contrôlé, et sans imposer la conservation du ligament croisé postérieur.

La prothèse concernée comprend, de manière connue du document EP-A-567 705, un élément fémoral, une embase tibiale et un plateau intermédiaire tels que précités, les condyles fémoraux présentant, vus dans le plan sagittal, une forme spiroïdale correspondant sensiblement à celle des condyles naturels, c'est-à-dire avec une réduction progressive de leurs rayons en direction des secteurs postérieurs, et le plateau intermédiaire présentant une éminence médiane qui forme une surface postérieure d'appui, tandis que l'élément fémoral présente un bossage médian à surface courbe, apte à prendre appui contre cette surface postérieure d'appui au cours du mouvement de l'articulation.

Ce bossage médian est conformé de manière à ne pas être en contact avec ladite surface postérieure d'appui au cours de ladite première partie mouvement, à entrer en contact avec cette surface postérieure d'appui lorsque ledit angle de flexion est atteint et à porter ensuite contre cette surface postérieure d'appui sur le reste de la course de flexion de l'articulation, en réalisant un "roll-back", c'est-à-dire un mouvement de roulement des condyles fémoraux par rapport à l'embase tibiale dans la direction postérieure.

Selon l'invention,
- les cavités glénoïdes du plateau intermédiaire et les condyles de l'élément fémoral sont conformés pour être congruents lorsque la prothèse est en extension et sur une première partie du mouvement de flexion de l'articulation s'étendant entre cette position d'extension et l'angle de flexion partir duquel la congruence des condyles avec les surfaces glénoïdes est perdue du fait de la forme spiroïdale des condyles ;
- les secteurs postérieurs des condyles et du bossage médian s'inscrivent dans des cercles de mêmes centres, et
- ladite surface postérieure d'appui présente une forme courbe correspondant à celle dudit bossage médian, de sorte que ce bossage prend appui contre cette surface postérieure d'appui sur une large surface durant l'ensemble de la deuxième partie de mouvement, consécutive à ladite première partie de mouvement.

La prothèse selon l'invention comprend ainsi des condyles fémoraux de forme spiroïdale ayant une congruence avec le plateau intermédiaire sur ladite première partie de mouvement de flexion. Cette forme et cette congruence permettent une bonne reproduction du mouvement de l'articulation naturelle et un bon contrôle du mouvement du fémur par rapport au tibia et inversement.

Au niveau dudit angle de flexion, le bossage médian vient au contact de ladite surface postérieure d'appui, selon un mouvement doux et progressif résultant des formes courbes complémentaires de ce bossage et de cette surface. Ce contact doux et progressif élimine tout choc de nature à solliciter les ancrages osseux.

A partir de cet angle de flexion, l'appui du bossage contre ladite surface postérieure d'appui supplée la perte de congruence des condyles fémoraux avec le plateau intermédiaire, ce qui permet de conserver le contrôle du mouvement de l'élément fémoral par rapport à l'embase tibiale et inversement ; le fait que les secteurs postérieurs des condyles et du bossage médian s'inscrivent dans des cercles de mêmes centres permet de réaliser le mouvement de "roll-back", qui permet non seulement de reproduire le mouvement anatomique mais également d'augmenter l'amplitude du mouvement de flexion de l'articulation et de ne pas générer de sollicitations néfastes sur les ancrages osseux en fin de ce mouvement de flexion.

Le plateau intermédiaire est avantageusement mobile par rapport à l'embase tibiale afin de limiter les sollicitations exercées sur ce plateau sur les ancrages osseux. Notamment, ce plateau peut être monté pivotant sur l'embase tibiale au moyen d'un pion que comprend le plateau intermédiaire ou l'embase, engagé dans une cavité correspondante aménagée respectivement dans l'embase ou le plateau intermédiaire.

Un exemple de réalisation de la prothèse selon l'invention est décrit ci-après en référence au dessin schématique annexé, dans lequel :
la figure 1 en est une vue sagittale, en coupe médiane antéro-postérieure ;
les figures 2, 3, 5, 7 et 9 sont des vues sagittales, en coupe médiane antéro-postérieure, de l'élément fémoral et du plateau intermédiaire de cette prothèse, respectivement en position d'extension de la prothèse et selon des angles de flexion de 30°, 60°, 90° et 125° ; et
les figures 4, 6, 8, 10 sont des vues du plateau intermédiaire en plan, montrant en hachures les zones de contact de l'élément fémoral avec ce plateau intermédiaire, respectivement dans les positions de cet élément fémoral et de plateau intermédiaire montrées sur les figures 2 et 3, 5, 7 et 9.

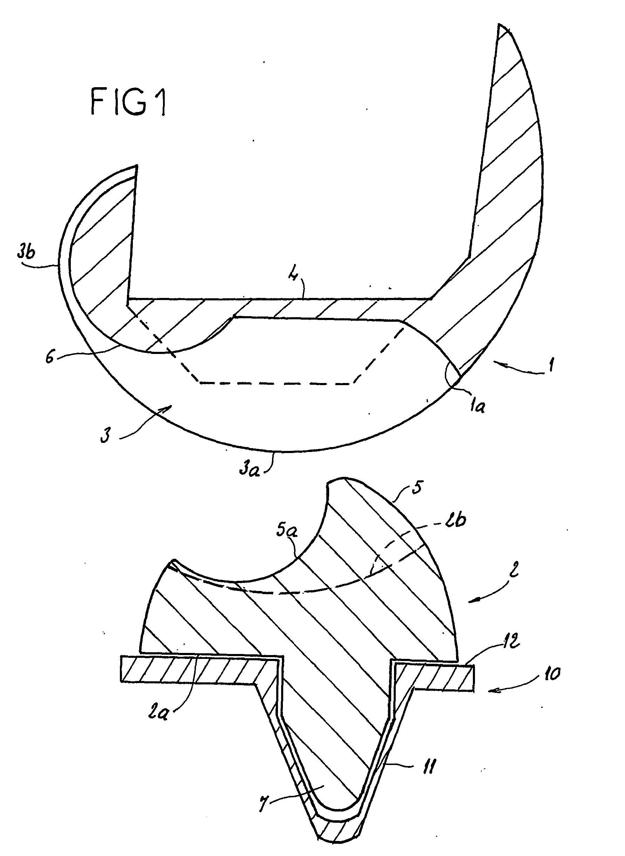

La figure 1 représente un élément fémoral 1, un plateau intermédiaire 2 et une embase tibiale 10 d'une prothèse totale du genou.

L'embase tibiale 10 présente une quille médullaire 11 permettant son ancrage dans le tibia, une plaque supérieure 12, destinée à recevoir le plateau intermédiaire 2, et une cavité cylindro-conique aménagée dans la quille 11.

L'élément fémoral 1 présente une forme recourbée propre à envelopper l'extrémité distale du fémur. Il présente des faces extérieures arrondies et des facettes internes destinées à prendre appui contre l'os, après résection adéquate de ce dernier. Pour son ancrage au fémur, il peut comprendre une tige médullaire et/ou des plots, non représentés.

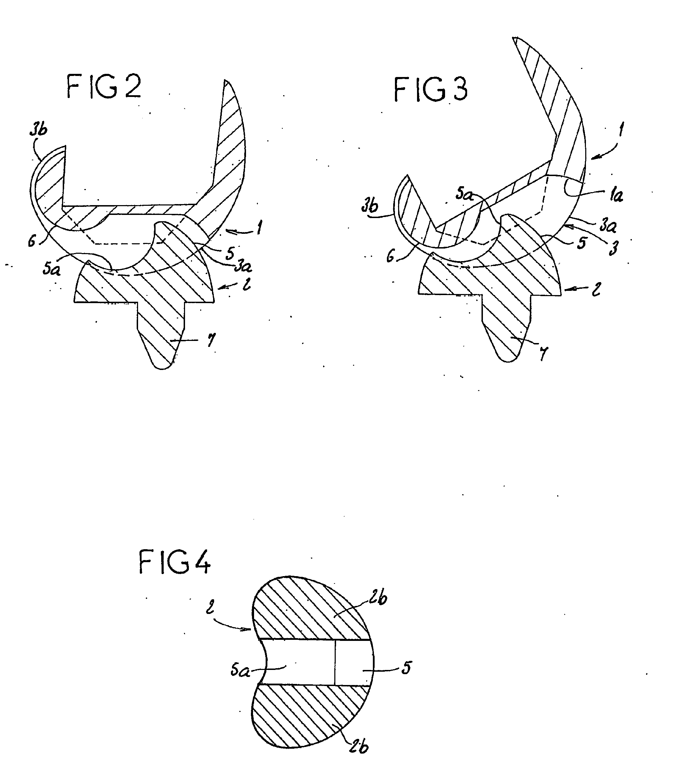

L'élément fémoral 1 forme des condyles latéraux 3 ayant, vus dans le plan sagittal, une forme spiroïdale correspondant sensiblement à celle des condyles naturels, c'est-à-dire avec une réduction progressive de leurs rayons en direction des secteurs postérieurs. Ainsi, la zone 3a médiane ou antéro-médiane de ces condyles 3, qui est portante entre 0 et environ 50° de flexion (cf. figures 2 et 3) présente, vue dans le plan sagittal, une forme substantiellement courbe d'un premier rayon tandis que le secteur postérieur 3b des condyles 3 s'inscrit substantiellement dans un cercle d'un deuxième rayon nettement inférieur audit premier rayon.

Dans le plan frontal, ces condyles 3 présentent une forme courbe convexe.

Sur la partie antérieure de l'élément fémoral 1, les condyles 3 délimitent entre eux une cavité allongée reproduisant substantiellement la trochlée anatomique. Au niveau de sa zone médiane, l'élément fémoral 1 comprend une cage médiane 4, allongée dans la direction antéro-postérieure, qui délimite une cavité ouverte vers le bas. Cette cavité est propre à recevoir une éminence 5 du plateau intermédiaire 2, décrite plus loin.

Comme cela apparaît en référence aux figures 1 et 2, l'élément fémoral 1 présente une face antérieure arrondie 1 a qui, en position d'hyperextension de l'articulation, prend appui contre la face antérieure arrondie de l'éminence 5.

Sur sa partie postérieure, l'élément fémoral 1 comprend un bossage médian arrondi 6. Comme le montrent les figures, le secteur postérieur de ce bossage 6 s'inscrit dans un cercle de mêmes centre que le cercle dans lequel s'inscrivent les secteurs postérieurs 3b des condyles 3.

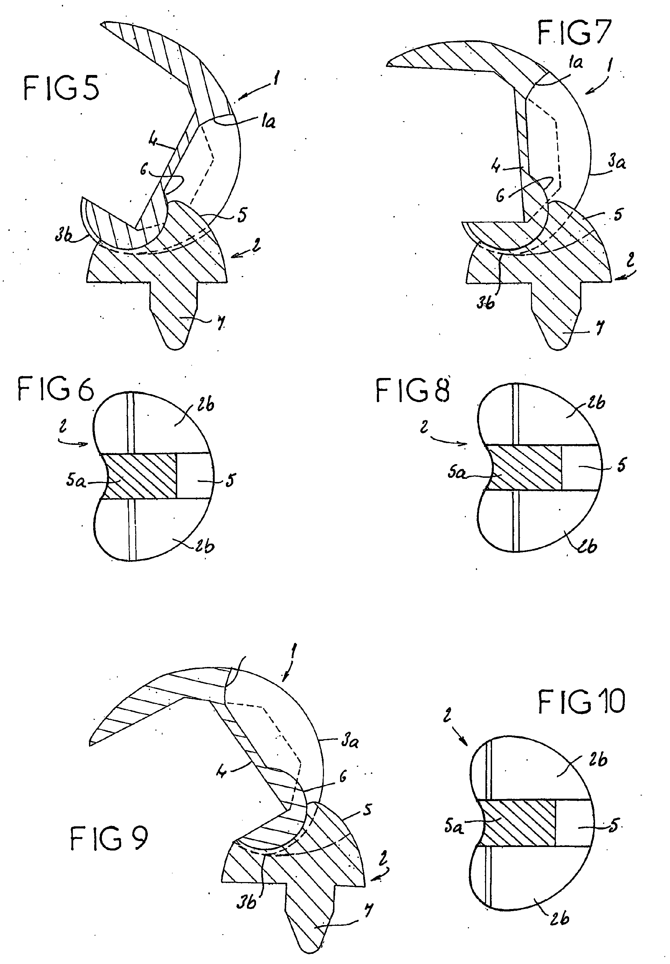

Le bossage 6 est disposé de manière à être à distance de ladite éminence 5 du plateau 2 en position d'extension de la prothèse (cf. figure 2) et en deça d'un angle de flexion de 60° (cf. figure 3). A partir de cet angle de flexion de 60° (cf. figure 5), ce bossage 6 vient en appui contre cette éminence 5 jusqu'à la position de totale flexion (cf. figures 7 et 9).

Dans le plan frontal, le bossage 6 présente une forme courbe et convexe.

Le plateau intermédiaire 2 est en matériau favorisant le glissement, notamment en polyéthylène à haute densité. Il présente une face inférieure 2a destinée à prendre appui contre la plaque supérieure 12 de l'embase tibiale 10 et des cavités glénoïdes 2b recevant les condyles 3 de l'élément fémoral 1. Ces cavités glénoïdes 2b ont une courbure au correspond à celle des zones 3a des condyles 3, avec lesquels elles sont par conséquent congruentes en deça d'un angle de flexion de 60°. Dans le plan frontal, elles ont une courbure qui correspond à celle des condyles 3 dans ce même plan.

Le plateau 2 comprend un pion cylindroconique 7 faisant saillie de dans sa face 2a, ayant un diamètre légèrement inférieur à celui de la cavité de la quille 11, qui peut recevoir ce pion 7 à pivotement.

Comme cela est visible sur les figures 2 et 3, les surfaces glénoïdes 2b et les condyles 3 sont conformés de manière à être congruents lorsque la prothèse est en extension ainsi que sur la partie du mouvement de flexion s'étendant en deça d'un angle de flexion de 60°. Le contact des condyles 3 avec les surfaces glénoïdes 2b s'étend sur des zones de sensiblement même surperficie que celle de ces surfaces glénoïdes 2b, comme le montre la figure 4.

A partir de l'angle de flexion de 60° et au-delà de cet angle, il apparaît progressivement un espace entre les condyles 3 et les surfaces 2b (cf. figure 5), résultant de la réduction de rayons précitée des condyles 3. La surface de contact entre les condyles 3 et les surfaces 2b se réduit ainsi progressivement, pour passer d'une étendue maximale en deça de 60° à un contact purement linéaire, comme le montre la figure 6.

L'éminence médiane 5 forme alors une surface postérieure d'appui 5a ayant globalement la même courbure que celle du bossage 6, prenant naissance environ au quart de la largeur du plateau 2 à partir du bord postérieur de ce plateau et ayant son sommet environ au tiers de la largeur du plateau 2 à partir du bord antérieur de ce plateau. Cette surface 5a présente une forme concave dans le plan frontal, dont la courbure correspond à celle du bossage 6 dans ce plan.

Comme cela apparaît sur les figures 5 à 10, au niveau d'un angle de 60° de flexion, le bossage 6 vient au contact de la surface 5a selon un mouvement doux et progressif résultant des formes courbes complémentaires de ce bossage 6 et de cette surface 5a. Ce contact doux et progressif élimine tout choc de nature à solliciter les ancrages osseux.

A partir de cet angle de 60° de flexion, la surface d'appui du bossage 6 contre la surface 5a supplée la perte de congruence des condyles 3 avec le plateau 2, comme le montrent les figures 6, 8 et 10, ce qui permet de conserver le contrôle du mouvement de l'élément fémoral 1 par rapport à l'embase tibiale 10 et inversement ; l'identité de forme du bossage 6 et des condyles 3 au niveau des secteurs postérieurs de ce bossage 6 et de ces condyles 3 permet de réaliser un mouvement de "roll-back", c'est-à-dire un mouvement de roulement des condyles 3 par rapport à l'embase tibiale dans la direction postérieure. Ce mouvement de roulement permet un recul des surfaces de contact linéaires des condyles 3 par rapport au plateau 2, sur une dizaine de milimètres dans l'exemple représenté, comme le montrent les figures 6, 8 et 10. Ce mouvement permet non seulement de reproduire le mouvement anatomique de l'articulation mais également d'augmenter l'amplitude du mouvement de flexion de l'articulation et de ne pas générer de sollicitations néfastes sur les ancrages osseux en fin de ce mouvement de flexion.

Ainsi qu'il apparaît de ce précède l'invention fournit une prothèse totale de genou à "postéro-stabilisation", présentant les avantages déterminants de conserver une congruence élevée entre l'élément fémoral et le plateau intermédiaire sur l'ensemble du mouvement de l'articulation, tout en permettant un mouvement de "roll-back" parfaitement contrôlé et sans imposer la conservation du ligament croisé postérieur.

Il va de soi que l'invention n'est pas limitée à la forme de réalisation décrite ci-dessus à titre d'exemple mais qu'elle en embrasse au contraire toutes les variantes de réalisation couvertes par les revendications ci-annexées. Notamment, les condyles 3 et le plateau 2 peuvent être conformés de telle sorte que le contact du bossage 6 avec l'éminence 4 intervienne à des angles de flexion autres que 60°, par exemple 30°, 40° ou même 80° ; le mouvement de "roll-back" peut aller, selon la conformation du bossage 6 et de l'éminence 5, de 2 à 20 mm.

## Revendications

1. - Prothèse totale du genou, comprenant :
- un élément fémoral (1) dont les condyles (3) présentent, vus dans le plan sagittal, une forme spiroïdale correspondant sensiblement à celle des condyles naturels, c'est-à-dire avec une réduction progressive de leurs rayons en direction des secteurs postérieurs, et qui comprend un bossage médian (6) à surface courbe ;
- une embase tibiale formant une plaque supérieure d'appui, et
- un plateau intermédiaire (2) destiné à prendre appui contre ladite plaque de l'embase tibiale et comprenant des cavités glénoïdes (2b) recevant les condyles (3) de l'élément fémoral (1), ce plateau intermédiaire (2) présentant une éminence médiane (5) qui forme une surface postérieure d'appui (5a) contre laquelle ledit bossage (6) est apte à prendre appui au cours du mouvement de l'articulation ;
- le bossage médian (6) est conformé de manière à ne pas être en contact avec ladite surface postérieure d'appui (5a) au cours de ladite première partie mouvement, à entrer en contact avec cette surface postérieure d'appui (5a) lorsque ledit angle de flexion est atteint et à porter ensuite contre cette surface postérieure d'appui (5a) sur le reste de la course de flexion de l'articulation, en réalisant un "roll-back", c'est-à-dire un mouvement de roulement des condyles (3) fémoraux par rapport à l'embase tibiale dans la direction postérieure ;
prothèse **caractérisée en ce que** :
- les cavités glénoïdes (2b) du plateau intermédiaire (2) et les condyles (3) de l'élément fémoral (1) sont conformés pour être congruents lorsque la prothèse est en extension et sur une première partie du mouvement de flexion de l'articulation s'étendant entre cette position d'extension et l'angle de flexion à partir duquel la congruence des condyles (3) avec les surfaces glénoïdes est perdue du fait de la forme spiroïdale des condyles (3) ;
- les secteurs postérieurs des condyles (3) et du bossage médian (6) s'inscrivent dans des cercles de mêmes centre, et
- ladite surface postérieure d'appui (5a) présente une forme courbe correspondant à celle dudit bossage (6) médian, de sorte que ce bossage (6) prend appui contre cette surface postérieure d'appui (5a) sur une large surface durant l'ensemble de la deuxième partie de mouvement, consécutive à ladite première partie de mouvement.

2. - Prothèse selon la revendication 1, **caractérisée en ce que** le plateau intermédiaire (2) est mobile par rapport à l'embase tibiale.

3. - Prothèse selon la revendication 2, **caractérisée en ce que** le plateau intermédiaire (2) est monté pivotant sur l'embase tibiale au moyen d'un pion que comprend le plateau intermédiaire ou l'embase tibiale, engagé dans une cavité correspondante (7) aménagée respectivement dans l'embase ou le plateau intermédiaire (2).

## Claims

1. - Complete knee prosthesis, comprising:
- a femoral element (1) which femoral condyles (3) have, seen in the sagittal plane, a spiral form corresponding substantially to that of the natural condyles, i.e. with gradual reduction of their radii towards posterior sectors, and which comprises a medial lobe (6) with a curved surface;
- a tibial seat forming an upper support plate, and
- a medial plateau (2) intended for resting against said upper support plate of the tibial seat and comprising glenoid cavities (2b) receiving the condyles (3) of the femoral element (1), said medial plateau (2) having a medial prominence (5) which forms a posterior support surface (5a), against which said lobe (6) is capable of resting during the movement of the joint;
- the medial lobe (6) is formed so as not to contact said posterior support surface (5a) during said first part of movement, but to contact said posterior support surface (5a) when said flexion angle is reached and then to carry the joint against this posterior support surface (5a) over the remainder of the flexion travel, while performing a "roll-back", i.e. a rolling movement of the femoral condyles (3) with respect to the tibial seat in the posterior direction,
prosthesis **characterised in that**:
- the glenoid cavities (2b) of the medial plateau (2) and the condyles (3) of the femoral element (1) are formed to be congruent when the prosthesis is in extension and on a first part of the flexion movement of the joint extending between this extension position and the angle of flexion from which the congruence of the condyles (3) with the glenoid surfaces is lost due to the spiral form of the condyles (3);
- the posterior sectors of the condyles (3) and of the medial lobe (6) inscribe circles of same centres, and
- said posterior support surface (5a) has a curved shape corresponding to that of said medial lobe (6), so that this lobe (6) rests against this posterior support surface (5a) over a wide surface throughout the second part of movement, consecutive to said first part of movement.

2. - Prosthesis according to claim 1, **characterised in that** the medial plateau (2) is mobile with respect to the tibial seat.

3. - Prosthesis according to claim 2, **characterised in that** the medial plateau (2) is mounted to pivot on the tibial seat by means of a stud included in the medial plateau (2) or the tibial seat, engaged in a corresponding cavity (7) provided respectively in the tibial seat or the medial plateau (2).

## Patentansprüche

1. Totalprothese des Knies, umfassend:
- ein femorales Element (1), dessen Gelenkköpfe (3), in sagittaler Ebene gesehen, eine spiraligen Form aufweisen, die etwa der Form der natürlichen Gelenkköpfe entspricht, das heißt, mit einer schrittweisen Reduzierung ihrer Radien in Richtung der hinteren Sektoren, und das eine mittlere Ausbauchung (6) mit gebogener Oberfläche umfasst;
- einen tibialen Ansatz, der eine obere Abstützplatte bildet, und
- ein Zwischenplateau (2), das dazu bestimmt ist, sich gegen die besagte Platte des tibialen Ansatzes zu stützen und Gelenkpfannenaufnahmen (2b) umfasst, die die Gelenkköpfe (3) des femoralen Elements (1) aufnehmen, wobei dieses Zwischenplateau eine mittlere Erhebung (5) aufweist, die eine hintere Abstützfläche (5a) bildet, gegen diese die besagte Ausbauchung (6) imstande ist, sich während der Gelenkbewegung abzustützen;
- wobei die mittlere Ausbauchung (6) derart aufgebaut ist, dass sie mit der besagten hinteren Abstützfläche (5a) während des besagten ersten Bewegungsabschnitts nicht im Kontakt ist, mit dieser hinteren Abstützfläche (5a) in Kontakt tritt, wenn der besagte Beugungswinkel erreicht ist und danach gegen diese hintere Abstützfläche (5a) über den Rest des Beugungswegs des Gelenks wirkt, durch Durchführung eines "roll-backs", das heißt, einer Rollbewegung der femoralen Gelenkköpfe (3) im Verhältnis zum tibialen Ansatz in die hintere Richtung,
wobei die Prothese **dadurch gekennzeichnet ist, dass**:
- die Gelenkpfannenaufnahmen (2b) des Zwischenplateaus (2) und die Gelenkköpfe (3) des femoralen Elements (1) ausgebildet sind, um kongruent zu sein, wenn die Prothese in Streckung ist und über einen ersten Abschnitt der Beugebewegung des Gelenks, der sich zwischen dieser Streckungsstellung und dem Beugungswinkel erstreckt, ab dem die Kongruenz der Gelenkköpfe (3) mit den Gelenkpfannenflächen aufgrund der spiraligen Form der Gelenkköpfe (3) verloren geht,
- sich die hinteren Sektoren der Gelenkköpfe (3) und der mittleren Ausbauchung (6) in Kreise gleicher Mitten einpassen, und
- die besagte hintere Abstützfläche (5a) eine gebogene Form aufweist, die der Form der besagten mittleren Ausbauchung (6) entspricht, so dass diese Ausbauchung (6) während des gesamten zweiten Bewegungsabschnitts, der dem besagten ersten Bewegungsabschnitt folgt, über eine breite Fläche gegen diese hintere Abstützfläche (5a) abstützt.

2. Prothese nach Anspruch 1, **dadurch gekennzeichnet, dass** das Zwischenplateau (2) im Verhältnis zum tibialen Ansatz beweglich ist.

3. Prothese nach Anspruch 2, **dadurch gekennzeichnet, dass** das Zwischenplateau (2) schwenkbar auf dem tibialen Ansatz mittels eines Stifts befestigt ist, den das Zwischenplateau oder der tibiale Ansatz umfasst, der in eine entsprechende Aufnahme (7) eingreift, der jeweils im Ansatz oder im Zwischenplateau (2) eingearbeitet ist.
